# EUROPEAN PATENT SPECIFICATION

(11) **EP 1 462 062 B1**
(45) Date of publication and mention of the grant of the patent: **04.10.2006**
(21) Application number: 04251568.4
(22) Date of filing: 18.03.2004
(51) Int. Cl.: A61B 17/122, A61B 17/08

(54) **Laminated surgical clip**
Laminierter chirurgischer Klip
Pince chirurgicale laminée

(30) Priority: 25.03.2003 US 396239
(43) Date of publication of application: 29.09.2004
(73) Proprietor: ETHICON ENDO-SURGERY, INC., Cincinnati, Ohio 45242 (US)
(72) Inventor: Francese, Jose Luis, Miami Springs, FL 33166 (US)
(74) Representative: Tunstall, Christopher Stephen

(56) References cited:
- GB-A- 293 301
- US-A- 4 275 813
- US-A1- 2002 198 549

## Description

### BACKGROUND OF THE INVENTION

### 1. Field of the Invention

This invention relates broadly to surgical devices. More particularly, this invention relates to a surgical clip for clamping and/or suturing, ducts, vessels, and other tissues, for anchoring a tissue, or for attaching a foreign body to a tissue.

### 2. State of the Art

Surgical clips are generally used to apply clamping force to ducts, vessels, and other tissues. In addition, surgical clips are particularly useful in controlling bleeding of a tissue in lieu of suturing or stapling where suturing or stapling is difficult. However, in certain circumstances, the bleeding tissue is lubricous, and applied clips often slip from the tissue and are dislodged, removing the necessary clamping force thereabout. This is particularly a problem when a clip is provided about tissue which is not a conduit of a size which can be completely surrounded by the clip. For example, it is very difficult to secure a clip about a small peripheral portion of ulcerated stomach tissue and therefore it is difficult to effect hemostasis of such bleeding tissue with a clip. Moreover, the problem is amplified when the clip used is very small.

In order to prevent dislodgement, a combination of a clip and a staple has been described in U.S. Patent No. 5,522,823 to Kuntz et al. In the Kuntz clip, one end portion of the clip is pierced through the tissue and captured in an eye of another end portion of the clip to secure the clip on the tissue. With the clip piercing the tissue, the likelihood that the clip will become inadvertently dislodged is greatly reduced.

While the Kuntz et al. clip represents a step forward, the disclosed clip is not particularly useful in endoscopic procedures. In particular, both the nature of the clip and the manner in which it is applied are complex. For example, in order to facilitate the bending of the clip through various configurations required of its applier, the clip has portions provided with at least four different widths as well as an eye opening. This complex clip structure is not practical for a clip which is to be used in a flexible endoscopy procedure in which the tools used are of very small diameter, e.g., 2-6 mm (0.08 - 0.24 inch). In addition, for endoscopic procedures it is highly desirable that multiple clips be able to be applied without removing the clip applier from its general location. The Kuntz et al. clip and applier, however, are not particularly adapted for applying multiple clips, as the Kuntz et al. clip does not stack, and the applier with which it is used holds a single clip at a time.

US 2002/0198549 discloses a clip suited to be applied by an endoscopic applicator. Multiple clips are disposed within the applicator for multiple applications

Another requirement for a clip is that it be relatively easily manufactured and at a reasonable cost. Otherwise, there will be resistance to its adoption for widespread use. In particular, it may be very difficult and costly to design a clip with desired features where such features are difficult to provide in a clip using a current cost-effective method of manufacture, such as stamping or bent forming. However, the cost-effective methods of manufacture cannot easily provide a clip in which the clip structure is not uniform across the width of the clip. Non-uniformity across the width of the clip may be desired to better permit the clip to engage tissue.

### SUMMARY OF THE INVENTION

It is therefore an object of the invention to provide a surgical clip which is adapted for use in minimally invasive surgery.

It is another object of the invention to provide a surgical clip which is non-uniform across its width.

It is an additional object of the invention to provide a surgical clip which is relatively easy to manufacture.

It is a further object of the invention to provide a surgical clip which remains secured to the tissue to which it is applied.

It is also an object of the invention to provide a surgical clip which can be applied in a flexible endoscopy setting.

It is yet another object of the invention to provide a surgical clip which can be used with rigid instruments operated through a port in the human body.

It is still another object of the invention to provide a surgical clip which can be used in open surgery.

It is still a further object of the invention to provide a surgical clip which is particularly adapted for use in an applier which holds a plurality of clips.

In accord with these objects, which will be discussed in detail below, a surgical clip comprises a laminated construction of subclip layers. In accord with a preferred embodiment, each subclip layer includes two substantially parallel arms and a bridge portion connecting the arms, and one of the arms includes a retainer element. In the laminate construction the subclip layers are alternated, such that the retainers are alternatingly provided on a top arm and then a bottom arm of the clip across the width of the clip. As such, the structure across the width of the clip is non-uniform.

The clip may also include structure to stack and even mechanically couple a train of the clips for use in a multifire clip applier device.

Additional objects and advantages of the invention will become apparent to those skilled in the art upon reference to the detailed description taken in conjunction with the provided figures.

### BRIEF DESCRIPTION OF THE DRAWINGS

Fig. 1 is a perspective view of a first embodiment of a laminated clip according to the invention;
Fig. 2 is a perspective exploded view of the laminated clip of Fig. 1;
Fig. 3 is a perspective view of a chain of mechanically linked stacked clips according to the invention; and
Fig. 4 is a perspective view of a second embodiment of a laminated clip according to the invention.

### DETAILED DESCRIPTION OF THE PREFERRED EMBODIMENTS

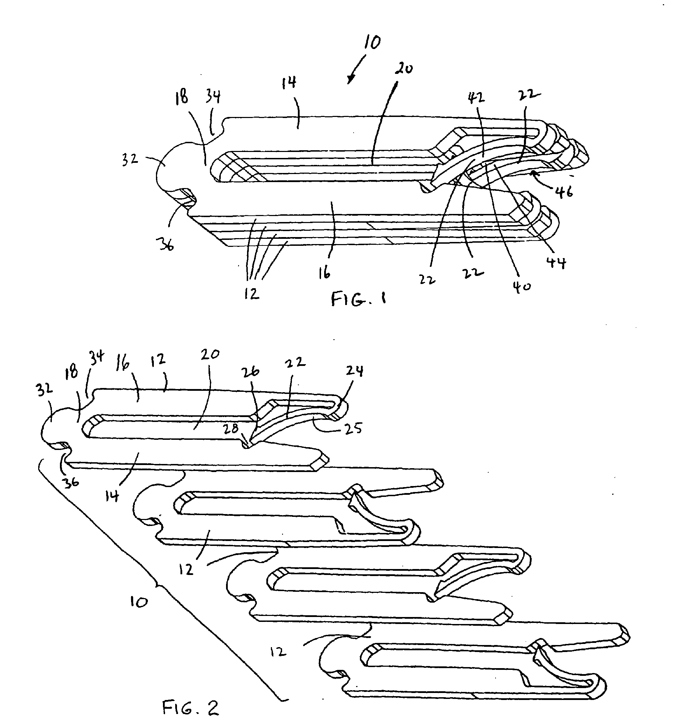

Turning now to Figs. 1 and 2, a surgical clip 10 according to the invention is shown. The clip 10 comprises a plurality of subclips, or layers, 12 which are laminated together to form composite clip 10. Four subclips 12 are shown, but fewer or more than four may be used. The lamination may be effected using adhesive, solder, welds, or any other suitable means by which to bond or fuse together the subclips 12, in a widthwise direction.

According to one preferred embodiment of the invention, each of the subclips 12 includes first and second substantially parallel arms 14, 16 and a bridge portion 18 therebetween such that the arms and bridge portion are in a generally U-shaped configuration with a space 20 defined therebetween.

For each of the subclips 12, the first arm 14 extends (or transitions) into a resilient retainer 22 which turns through slightly more than 180° at a distalmost extension 24 of the arm, and then curves toward the second arm 14 to define a first catch portion 25. The retainer 22 preferably terminates in a barbed tip 26 and interrupts access to the space 20 between the arms. The second arm 16 defines a notch 28 towards which the tip 26 is directed and may be partially received. The bridge portion 18 includes a generally omega-shaped projection 32 and recesses 34, 36 defined between the projection 32 and the first arm 14 and between the projection 32 and the second arm 16.

The subclips are organized such that the retainers 22 of the subclips 12 alternatingly extend upward and downward. That is, adjacent subclips 12 are offset by the width of a clip and rotated by 180°. Thus, in the assembled laminated clip 10, every other retainer extends from an upper first arm toward a lower second arm, and the alternating retainers extend from a lower first arm toward an upper second arm. As such, the laminated clip 10 includes negative space 40 between two portions of positive space (i.e., structure), e.g., 42, 44, across the width of the clip 10, and similarly positive space 42 between two areas of negative space, e.g., 40, 46 across the width of the clip 10 (Fig. 1).

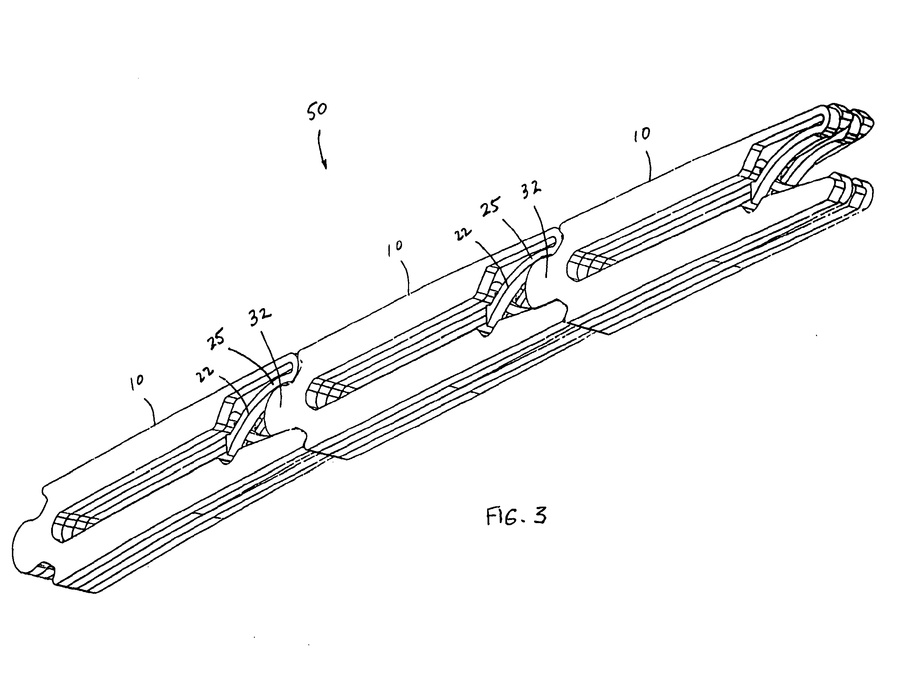

Referring to Fig. 3, the projection 32, recesses 34, 36, and the first catch portion 25 of the first arm 14 cooperate to define a mechanical interlock such that, as described in more detail below, a plurality of laminated clips 10 may be coupled together in a linear arrangement. That is, when longitudinally arranged, the projection 32 of a laminated relatively distal clip 10 extends between the first and second arms 14, 16 of the laminated clip 10 and into the catch portions 25 thereof, with the end of the first arm 14 extending around the projection 32 and into recess 34 of the laminated clip, while the end of the second arm 16 extends around the projection 32 and into recess 36 to couple the clips together in a clip train 50. When the clips 10 are provided in a clip chamber of a clip applier instrument and such chamber is sized or otherwise configured to restrict movement of the first and second arms away from each, the projection 32 of a relatively distal clip is captured and retained by the adjacent relatively proximal clip. As such, the clips do not separate from each other while in a clip chamber.

In use, a clip chamber of a clip applier is loaded with a train of the clips 10. The clip applier includes a distal end having a jaw assembly and an exit through which clips can be advanced from the clip chamber to between the jaws. Flexible endoscopic clip appliers having such features are generally disclosed in U.S. Patent Nos. 6,716,226, filed June 25, 2001, and 6,824,548, filed Dec. 6, 2001. The clip applier is inserted through an endoscope into the human body, and the jaw assembly is moved adjacent the target tissue identified for receiving a clip and operated to clamp and compress the target tissue. Once the tissue is compressed, preferably to a thickness approximating the space 20 between the first and second arms 14, 16, the distalmost clip of the clip train 50 is advanced through the jaw assembly and over the compressed tissue. The multiple retainers 22 of the laminated clip 10 are resiliently deformed during advancement such that the retainers are bent to provide access to the space 20. The clip is advanced over the tissue, preferably until the tissue is either seated against the bridge 18 of the clip and/or fully resides proximal of the ends 26 of the retainers 22 so that the retainers may reform to again interrupt the space. In accord with a preferred embodiment, the arms 14, 16 and bridge 18 are relatively stiff, such that the arms and bridge retain their shape and are not plastically deformed during application over tissue. That is, any expansion of the clip between the arms during application is minimal and elastic. As such, it is appreciated that one preferred embodiment of the clip 10 is passive in that the clip is not actively deformed by the clip applier during application.

Regardless of the exact position of the clip on the tissue, movement of the clip 10 in a release direction is prohibited by the retainers 22 which are directed to pierce or contact tissue if the clip is urged in a release direction. After attaching the clip to tissue, the jaw assembly is opened to remove clamping force from the tissue.

When the distalmost clip is attached to the tissue, it is positioned within the jaw assembly such that the end of the arms of the proximally adjacent clip extend partially out of the clip chamber. Therefore, the end of the arms of the proximally adjacent clip are not constrained by the clip chamber. As such, after the distalmost clip is attached to tissue and the jaw assembly is opened, the clip applier (or just the clip train) can be retracted to subject the clip train 50 to a tensile force. The tensile force urges the unconstrained arms of the clip proximally adjacent to the distalmost clip to slightly flex and thereby release the distalmost clip to deploy the clip. The clip train is then slightly retracted in preparation of application of a subsequent clip.

The clips are particularly suitable for use in a flexible endoscopic clip applier, as described, though they may be also used in rigid instruments in both laparoscopic and open surgery. For endoscopic uses, the clips are preferably manufactured in the small sizes necessary for such minimally invasive procedures, e.g., a height of 1 - 3 mm (0.04 - 0.12 inch) between the outer surfaces of the first and second arms. For other uses, the clips may be appropriately sized. The clips, in all sizes, preferably, though not necessarily, have a length to height ratio of between approximately 2.5 and 5, and most preferably of approximately 3.4. In a preferred embodiment, each subclip laminate layer is approximately 0.2 mm (0.008 inch) and the clip comprises four layers for a total thickness of 0.8 mm (0.032 inch). However, two to ten or more clips may be laminated together to provide composite clips of 0.4 - 2 mm (0.016 inch to 0.080 inch) or more in width. Provided that the fusing or bonding method provides at least as much strength as the original material, the strength of the composite clip should be the same as a unitarily formed clip.

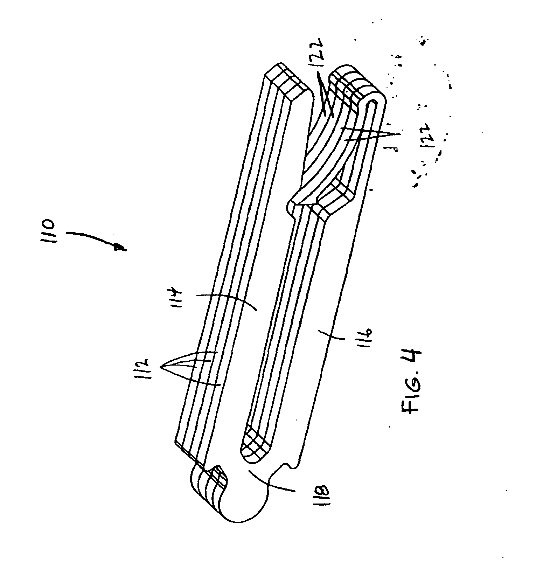

It is appreciated that numerous other configurations of the subclips can be used to comprise a composite laminated clip in accord with the invention. For example, referring to Fig. 4, another embodiment of a composite clip 110 is shown. The clip 110 includes four subclips 112, each of a design similar to subclip 12, but which are laminated together in a common orientation. That is, the retainers 122 all extend adjacent to each other in the same direction. In clip 110, while the arms 114, 116 and bridges 118 of adjacent subclips 112 are laminated together, the retainers 122 are preferably not laminated together, such that each retainer 122 is independently movable relative to the others. As such, each of the individual retainers has a relatively small cross-sectional area relative to an otherwise unitary retainer extending across the width composite clip. Thus, the independently movable retainers have increased flexibility. Moreover, a retainer with a relatively larger height can be more easily manufactured, and the smaller independently movable retainers 122 of the subclips 112 maintain a high degree of resiliency even with the increased retainer height.

In addition, any of the clips described in co-owned and copending U.S. Paten No. 6,716,226, can be laminated together in accordance with the present invention. More particularly, the clips described in U.S. Patent No. 6,716,226 are actively deformable. That is, such clips having retainers that are plastically deformed by the clip applier during application to tissue. In this manner, the retainers of such clips can be directed to pierce tissue to facilitate retention. In accord with the present invention, the subclips may be configured within the laminated composite clip 10 to alternatingly be provided on upper and lower arms of the subclips such that the composite clip has deformable retainers on both top and bottom arms of the clip. Furthermore, it is also noted that where a plastically deformable retainer is used, such retainer may be an extension of one of the arms, and that such extension may have a relatively larger length to height ratio, e.g., greater than 5.

The subclips according to the various embodiments of the invention are preferably made from a unitary piece of metal or metal alloy, such as titanium, titanium alloy, stainless steel, tantalum, platinum, other high Z (substantially radiopaque) materials, nickel-titanium alloy, martensitic alloy, or plastic, although other suitable biocompatible materials may be used.

There have been described and illustrated herein embodiments of a laminated surgical clip and a method of manufacturing a laminated clip. While particular embodiments of the invention have been described, it is not intended that the invention be limited thereto, as it is intended that the invention be as broad in scope as the art will allow and that the specification be read likewise. Thus, while the clip is particularly adapted for manufacture in the small size necessary for flexible endoscopy, it will be appreciated that the clip may be made in other sizes as well. Furthermore, aspects of the various embodiments may be combined in yet other embodiments. Moreover, other stacking structure and other retainer configurations are shown in the applications incorporated by reference, and such stacking structure may be provided in combination with any of the retainer structures. It will therefore be appreciated by those skilled in the art that yet other modifications could be made to the provided invention without deviating from the scope of the claims.

## Claims

1. A surgical clip, comprising:
a plurality of substantially flat subclip layers (12) laminated together to form a composite clip (10), said subclip layers adapted to remain laminated together during implantation.

2. A surgical clip according to claim 1, wherein:
said laminated subclip layers (12) are bonded together.

3. A surgical clip according to claim 1, wherein:
said laminated subclip layers (12) are fused together.

4. A surgical clip according to claim 1, wherein:
at least two of said subclip layers (12) include a deformable retainer element (22), said retainer element (22) on one of said at least two subclips layers (12) being in a descending orientation, and said retainer element (22) on another of said at least two subclip layers being in an ascending orientation.

5. A surgical clip according to claim 4, wherein:
said retainer (22) is elastically deformable.

6. A surgical clip according to claim 4, wherein:
said retainer (22) is plastically deformable.

7. A surgical clip according to claim 1, wherein:
said laminated clip defines a first arm (14), a second arm (16), a bridge portion (18) coupling said first (14) and second arms (16), and a plurality of retainers (22) coupled to said first (14) and second (16) arms.

8. A surgical clip according to claim 7, wherein:
said first (14) and second (16) arms are substantially parallel to each other.

9. A surgical clip according to claim 7, wherein:
said first (14) and second (16) arms are substantially stiff.

10. A surgical clip according to claim 7, wherein:
said plurality of retainers (22) of said laminated clip (10) are movable relative to each other.

11. A surgical clip according to claim 10, wherein:
said plurality of retainers (22) all extend in a substantially same direction.

12. A surgical clip according to claim 1, wherein:
each said subclip layer (12) includes a first arm (14), a second arm (16), and a bridge portion (18) coupling said first and second arms.

13. A surgical clip according to claim 12, wherein:
at least two of said subclip layers (12) each include a retainer (22) coupled to one of said first (14) and second (16) arms.

14. A surgical clip according to claim 13, wherein:
each said retainer (22) includes a barbed tip (26).

15. A surgical clip according to claim 1, further comprising:
means (32,34) for stacking said clip with a proximally adjacent clip (10) and a distally adjacent clip (10).

16. A surgical clip according to claim 15, wherein:
said means (32,34) for stacking mechanically interlocks said clip with a proximally adjacent clip (10) and a distally adjacent clip (10).

17. A surgical clip according to claim 1, wherein:
said clip (10) has a width, and said clip is non-uniform across said width.

18. A surgical clip according to claim 17, wherein:
said clip (10) has a width, and said clip defines negative space between two portions of structure across said width.

19. A surgical clip according to claim 1, wherein:
each said subclip layers (12) has a common shape, and adjacent subclips layers (12) are offset from each other and rotated by 180°.

20. A surgical clip according to claim 1, wherein:
said clip (10) is comprised of two to ten subclips (12).

21. A surgical clip according to claim 1, wherein:
said clip (10) has a thickness between 0.4-2mm (0.016 in to 0.080 in).

## Patentansprüche

1. Chirurgischer Clip, mit:
einer Vielzahl im wesentlichen ebener Teilclipschichten (12), die aneinander laminiert sind, um einen Verbundclip (10) zu bilden, wobei die Teilclipschichten so ausgelegt sind, daß sie während des Implantierens aneinander laminiert bleiben.

2. Chirurgischer Clip nach Anspruch 1, bei dem:
die laminierten Teilclipschichten (12) miteinander verbunden sind.

3. Chirurgischer Clip nach Anspruch 1, bei dem:
die laminierten Teilclipschichten (12) miteinander verschmolzen sind.

4. Chirurgischer Clip nach Anspruch 1, bei dem:
wenigstens zwei der Teilclipschichten (12) ein deformierbares Halteelement (22) umfassen, wobei das Halteelement (22) auf einer der wenigstens zwei Teilclipschichten (12) in einer abhängenden Ausrichtung ist und das Halteelement (22) auf einer weiteren der wenigstens zwei Teilclipschichten in einer aufragenden Ausrichtung ist.

5. Chirurgischer Clip nach Anspruch 4, bei dem:
das Halteelement (22) elastisch deformierbar ist.

6. Chirurgischer Clip nach Anspruch 4, bei dem:
das Halteelement (22) plastisch deformierbar ist.

7. Chirurgischer Clip nach Anspruch 1, bei dem:
der laminierte Clip einen ersten Arm (14), einen zweiten Arm (16), einen Brückabschnitt (18), der den ersten (14) und den zweiten Arm (16) koppelt, und eine Vielzahl von Halteelementen (22), die mit dem ersten (14) und dem zweiten (16) Arm gekoppelt sind, definiert.

8. Chirurgischer Clip nach Anspruch 7, bei dem:
der erste (14) und der zweite (16) Arm im wesentlichen parallel zueinander sind.

9. Chirurgischer Clip nach Anspruch 7, bei dem:
der erste (14) und der zweite (16) arm im wesentlichen steif sind.

10. Chirurgischer Clip nach Anspruch 7, bei dem:
die Vielzahl der Halteelemente (22) des laminierten Clips (10) relativ zueinander bewegbar sind.

11. Chirurgischer Clip nach Anspruch 10, bei dem:
die Vielzahl der Halteelemente (22) sich alle in im wesentlichen dieselbe Richtung erstrecken.

12. Chirurgischer Clip nach Anspruch 1, bei dem:
jede Teilclipschicht (12) einen ersten Arm (14), einen zweiten Arm (16) und einen Brückenabschnitt (18), der den ersten und den zweiten Arm koppelt, umfaßt.

13. Chirurgischer Clip nach Anspruch 12, bei dem:
wenigstens zwei der Teilclipschichten (14) jeweils ein Halteelement (22), das entweder mit dem ersten (14) oder dem zweiten (16) Arm gekoppelt ist, umfassen.

14. Chirurgischer Clip nach Anspruch 13, bei dem:
jedes Halteelement (22) eine Spitze (26) mit Widerhaken umfaßt.

15. Chirurgischer Clip nach Anspruch 1, der weiter aufweist:
eine Einrichtung (32, 34) zum Stapeln des Clips mit einem proximal benachbarten Clip (10) und einem distal benachbarten Clip (10).

16. Chirurgischer Clip nach Anspruch 15, bei dem:
die Einrichtung (32, 34) zum Stapeln mechanisch den Clip mit einem proximal benachbarten Clip (10) und einem distal benachbarten Clip (10) verriegelt.

17. Chirurgischer Clip nach Anspruch 1, bei dem:
der Clip (10) eine Breite hat und der Clip über die Breite nicht gleichförmig ist.

18. Chirurgischer Clip nach Anspruch 17, bei dem:
der Clip (10) eine Breite hat und der Clip negativen Raum zwischen zwei Teilen der Struktur über die Breite definiert.

19. Chirurgischer Clip nach Anspruch 1, bei dem:
jede der Teilclipschichten (12) eine gemeinsame Form hat und benachbarte Teilclipschichten (12) voneinander versetzt und um 180° gedreht sind.

20. Chirurgischer Clip nach Anspruch 1, bei dem:
der Clip (10) aus zwei bis zehn Teilclips (12) besteht.

21. Chirurgischer Clip nach Anspruch 1, bei dem:
der Clip (10) eine Dicke zwischen 0.4 - 2 mm (0.016 Zoll bis 0.080 Zoll) hat.

## Revendications

1. Pince chirurgicale, comprenant:
plusieurs couches de sous-pinces sensiblement plates (12) laminées ensemble pour former une pince composite, lesdites couches de sous-pinces étant aptes à rester laminées ensemble pendant l'implantation.

2. Pince chirurgicale selon la revendication 1, où lesdites couches de sous-pinces laminées (12) sont liées les unes aux autres.

3. Pince chirurgicale selon la revendication 1, où lesdites couches de sous-pinces laminées 12 sont assemblées par fusion.

4. Pince chirurgicale selon la revendication 1, où au moins deux desdites couches de sous-pinces (12) comprennent un élément de retenue déformable (22), ledit élément de retenue (22) sur une desdites au moins deux couches de sous-pinces (12) étant dans une orientation descendante, et ledit élément de retenue (22) sur une autre desdites au moins deux couches de sous-pinces étant dans une orientation ascendante.

5. Pince chirurgicale selon la revendication 4, où ledit élément de retenue (22) est déformable élastiquement.

6. Pince chirurgicale selon la revendication 4, où ledit élément de retenue (22) est plastiquement déformable.

7. Pince chirurgicale selon la revendication 1, où ladite pince laminée définit un premier bras (14), un second bras (16), une portion de dos (18) reliant lesdits premier (14) et second bras (16), et plusieurs éléments de retenue (22) couplés auxdits premier (14) et second (16) bras.

8. Pince chirurgicale selon la revendication 7, où lesdits premier (14) et second (16) bras sont sensiblement parallèles l'un à l'autre.

9. Pince chirurgicale selon la revendication 7, où lesdits premier (14) et second (16) bras sont sensiblement rigides.

10. Pince chirurgicale selon la revendication 7, où plusieurs éléments de retenue (22) de ladite pince laminée (10) sont déplaçables les uns par rapport aux autres.

11. Pince chirurgicale selon la revendication 10, où plusieurs éléments de retenue précitée (22) s'étendent tous sensiblement dans la même direction.

12. Pince chirurgicale selon la revendication 1, où chaque couche de sous-pinces précitée (12) comprend un premier bras (14), un second bras (16) et une portion de dos (18) reliant lesdits premier et second bras.

13. Pince chirurgicale selon la revendication 12, où au moins deux desdites couches de sous-pinces (12) comprennent chacune un élément de retenue (22) couplé à l'un desdits premier (14) et second (16) bras.

14. Pince chirurgicale selon la revendication 13, où chaque élément de retenue précité (22) comprend une pointe à barbe (26).

15. Pince chirurgicale selon la revendication 1, comprenant en outre un moyen (32,34) pour empiler ladite pince sur une pince proximalement adjacente (10) et une pince distalement adjacente (10).

16. Pince chirurgicale selon la revendication 15, où ledit moyen (32,34) pour l'empilage interverrouille mécaniquement ladite pince avec une pince proximalement adjacente (10) et une pince distalement adjacente (10).

17. Pince chirurgicale selon la revendication 1, où ladite pince (10) a une largeur, et ladite pince est non-uniforme sur ladite largeur.

18. Pince chirurgicale selon la revendication 17, où ladite pince (10) a une largeur, et ladite pince définit un espace négatif entre deux portions de structure sur ladite largeur.

19. Pince chirurgicale selon la revendication 1, où chacune desdites couches de sous-pinces (12) a une forme commune, et des couches de sous-pinces adjacentes (12) sont décalées l'une de l'autre et tournées selon 180°.

20. Pince chirurgicale selon la revendication 1, où ladite pince (10) est constituée de deux à dix sous-pinces (12).

21. Pince chirurgicale selon la revendication 1, où ladite pince (10) a une épaisseur entre 0,4-2mm (0,016 in à 0,080 in).
